# EUROPEAN PATENT APPLICATION

(11) **EP 3 960 203 A1**
(43) Date of publication of application: **02.03.2022**
(21) Application number: 20192755.5
(22) Date of filing: 25.08.2020
(51) Int. Cl.: A61K 47/58

(54) **COMPLEXES FOR THE DELIVERY OF PROTEINACEOUS AGENTS**

(71) Applicant: Université de Liège, 4000 Liège (BE)
(72) Inventor: GRANDFILS, Christian, 4020 Liège (BE); SEVRIN, Chantal, 4020 Liège (BE)
(74) Representative: Peel, James Peter

(57) **Abstract**

The invention provides a complex comprising at least one proteinaceous agent, an ionic polymer comprising a repetitive unit of formula (I): 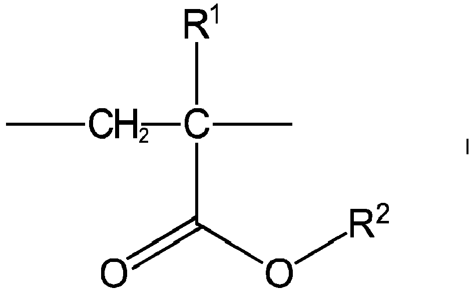 wherein R¹ represents a hydrogen atom or a straight or branched chain alkyl group, preferably a straight or branched chain alkyl group comprising from 1 to 6 carbon atoms, for example a methyl group; R² represents a straight or branched chain alkyl group which is substituted by a group which may have a positive charge at a physiological pH; and optionally a surfactant; a complex for use in a method of medical treatment; a pharmaceutical composition

## Description

### Technical field of the invention

The invention concerns a formulation for the delivery of a proteinaceous agent.

### Background of the invention

Rapid progress in peptide and protein production technologies has resulted in the availability of a wide variety of protein- and peptide-based drugs targeting poorly controlled diseases. Several hundred protein drugs are currently either on the market, either undergoing clinical trials. However, the use of these drugs may be severely limited by the fragile structure of the biomolecules and the body's ability to rapidly remove biomolecules from the bloodstream.

Parenteral and, to a certain extent, oral routes of administration of peptides and proteins are dominated by micro- and nano-particulate systems wherein the drug is encapsulated in a solid polymer. By contrast, the bioavailability of peptides and proteins from non-parenteral mucosal routes is, in general, poor when compared with the parenteral route. Critical issues associated with Peptide/Protein (P/P) delivery via alternative administration routes include:
- P/Ps are high molecular weight biopolymers. Due to their large molecular weight and, thus, size, they show poor permeability through the various mucosal surfaces and biological membranes.
- Many P/P drugs are efficacious due to their tertiary structure. However, their tertiary structure can be lost under various physical and chemical environments, resulting in their denaturation or degradation with consequent loss of their biological activity, hence, making them inherently unstable.
- Many P/P drugs have very short biological half-lives in vivo due to their rapid clearance in the liver and other body tissues by proteolytic enzymes.
- As P/P drugs have very specific actions and are highly potent, their precise clinical dosing is of utmost importance.

Previous studies have demonstrated that the biodistribution and pharmacokinetic profiles of the P/P drugs when they are incorporated in nanocarriers can be significantly improved compared to the respective profiles of the free P/P drugs. Known strategies include primarily the so-called "pegylation" technique which consists in a chemical grafting of polyethylene glycol (PEG) to the peptide but which leads to a risk of conformation change and a loss of its biological activity; secondly the inclusion of compounds within a molecular cage such as cyclodextrin. The latter strategy is however not suitable for all P/P drugs as it is too small for P/P drugs requiring a molecular cage of a diameter as large as from 1-2 nm to at least 10 nm. Therefore, synthetic polymers which typically can provide a cage having a hydrodynamic diameter of several nm or ten of nm are preferred. Vetter et al. disclosed the use of water-soluble hyperbranched polymer attached to a core and to the bioactive protein (Protein-proteophore complexes, WO 2005034909A2). According to their approach, the protein/polymer complexes are stabilized with means of a substantially non-enzymatically cleavable linker. Alpar et al. reported polycationic carbohydrate such as chitosan as immunostimulants in vaccine compositions, in particular in particles in the form of microparticles or liposomes (Polycationic carbohydrates as immunostimulants in vaccines, WO200056361A2). Illum et al. disclosed the preparation of microspheres which may be of starch, gelatin, dextran, collagen or albumin loaded with peptides, such as insulin, and antigenic vaccine ingredients in order to promote their absorption upon their administration in the nasal cavity (Small particle compositions for intranasal drug delivery US5707644A).

Other authors have reported Block lonomer Complexes (BIC) to nanoencapsulate drug entities such as Kabanov et al. (Compositions for protein delivery and methods of use thereof, WO2008141155 and Polyelectrolyte complexes for delivery of agents to the CNS, WO2017136376). These authors used block copolymers which are conjugates of least two different polymer segments: a non-ionic segment and an ionic segment. The incorporation of a biological agent such as a protein is assured by the formation of a complex with the ionic segment of the block copolymer. The non-ionic segment is used to keep the complex in solution. According to these authors, this two-segment structure is required to promote the interaction between the block copolymer and the biological agent, but also to stabilize in aqueous medium the hydrophobic domains resulting from the neutralisation of the ionic segment by the charges of the biological agent. Hence, the complex disclosed is a core-shell like nanostructure in which the biological agent is entrapped.

### Description of the invention

Surprisingly, it has been found it is possible to prepare a "Poly Amphiphilic Electrolyte Complex" (PAEC) with a proteinaceous compound with careful selection of ionic polymer with unique characteristics as defined below. The ionic polymer is disclosed in WO2015/028286 (Université de Liege) as a medicament for use in the treatment of a heparin overdose. The Examples of WO2015/028286 include *in vitro* formation of polyelectrolyte complexes to test the heparin neutralisation effectiveness. The polyelectrolyte complexes were formed by heparin and an ionic polymer which is a poly(dimethyl amino) ethyl methacrylate having a relative number-average molecular weight of 8,000 or 15,000, in PBS or in plasma. The disclosure of WO2015/028286 is incorporated herein by reference.

According to the invention there is provided a complex comprising at least one proteinaceous agent, an ionic polymer comprising a repetitive unit of formula (I): 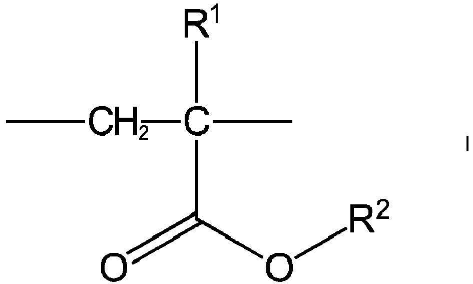 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which optionally has a positive charge at a physiological pH; and optionally a surfactant; with the proviso that when the at least one proteinaceous agent is heparin and when the complex does not include a surfactant, the ionic polymer is not a poly (dimethyl amino) ethyl methacrylate having a relative number-average molecular weight of 8,000 or 15,000.

According to the invention there is further provided a complex for use in a method of medical treatment wherein the complex comprises at least one medically or veterinary active proteinaceous agent, an ionic polymer comprising a repetitive unit of formula (I): 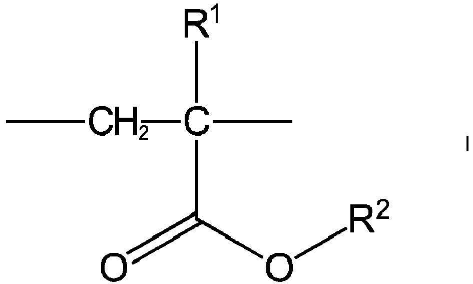 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which optionally has a positive charge at a physiological pH; or a copolymer thereof; and optionally a surfactant.

According to the invention there is also provided a pharmaceutical composition comprising a carrier and a complex comprising at least one proteinaceous agent, an ionic polymer comprising a repetitive unit of formula (I); and optionally a surfactant; with the proviso that when the carrier is phosphate-buffered saline, and when the at least one proteinaceous agent is heparin, and when the complex does not include a surfactant, the ionic polymer is not a poly (dimethyl amino) ethyl methacrylate having a relative number-average molecular weight of 8,000 or 15,000.

According to the invention there is further provided a pharmaceutical composition for use in a method of medical treatment wherein the composition comprises a carrier and a complex comprising at least one medically- or veterinary- active proteinaceous agent, an ionic polymer comprising a repetitive unit of formula (I): 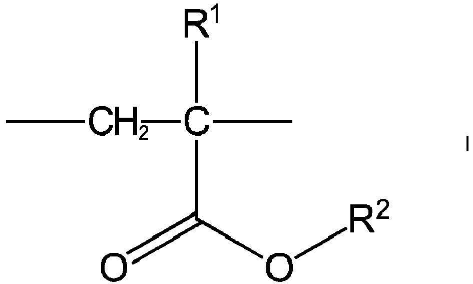 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which optionally has a positive charge at a physiological pH; or a copolymer thereof; and optionally a surfactant.

According to the invention there is also provided a method of medical treatment which method includes a step of administering to a human or animal in need of such treatment an effective amount of a complex which comprises at least one medically active proteinaceous agent, an ionic polymer comprising a repetitive unit of formula (I): 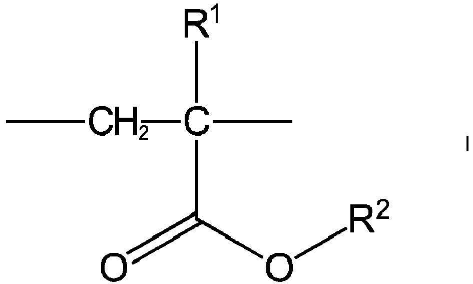 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which optionally has a positive charge at a physiological pH; or a copolymer thereof; and optionally a surfactant.

In some embodiments, the ionic polymer of formula (I) may be prepared by living radical polymerization such as atom-transfer radical polymerization (ATRP), to control the macromolecular parameters of the polymer. In some embodiments, the ionic polymer may have a molecular weight which is from 1 kDa to 100 kDa.

In some embodiments, the ionic polymer may have a mean charge density which is the proportion of the number of positively charged repetitive units to the number of repetitive units. In some embodiments, the mean charge density of the ionic polymer of formula (I) at physiological pH may range from 5 to 100 %, for example a mean charge density of from 10 to 50 %. Several macromolecular architectures can be tailored by living radical polymerisation in order to play on the flexibility of polymer chain, on the separation between segments bearing charges and neutral segments, and on the global hydrophilic/hydrophobic ratio of the polymer.

The main role of the ionic polymer is to promote the formation of a poly amphiphilic electrolyte complex (PAEC) with the at least one proteinaceous agent in order to protect the proteinaceous agent from the biological environment, but also to enhance adhesion of the PAEC to biological mucosa and to cross the biological barriers more easily. Advantageously the ionic polymer enables the reversibility of its interaction with the at least one proteinaceous agent in such a way that according to physiological trigger(s), the proteinaceous agent can be released under an active form, according to a specific release rate and the right biological site. In some embodiments, the physiological trigger can be related to a change in temperature and/or a change in local pH.

In some embodiments, the ionic polymer can be made from one single repetitive unit of formula (I), or alternatively the repetitive unit of formula (I) can be combined with other monomers or other polymer or oligomeric sequences. In some embodiments, the ionic polymer may consist essentially of repetitive units of formula (I) wherein at least some repetitive units of formula (I) bear a charge. In some embodiments, the ionic polymer may comprise (or consist of) repetitive units of formula (I) wherein at least some repetitive units of formula (I) bear a charge. In some embodiments, the ionic polymer may be an ionic homopolymer which is at least partially charged. In some embodiments, the charge may be a positive charge, in particular a positive charge at physiological pH. In some embodiments, the ionic polymer may be a cationic homopolymer. In some embodiments, the ionic polymer is an ionic polymer of formula: 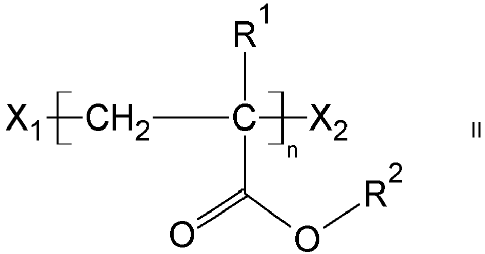 wherein X₁ and X₂ each respectively represent the alpha and omega end groups of the polymer; R¹ and R² are each as defined for the repetitive unit of formula (I); and n represents the number of repetitive units of the ionic polymer.

In some embodiments, the ionic polymer may be characterized by an intermediate solubility such that it is soluble in various solvents, including an aqueous medium, but also a chlorinated solvent (such as methylene chloride or chloroform) heterocyclic ethers such as tetrahydrofuran, but also dimethylsulfoxide, dimethylacetamide or dimethylformamide. If other type of polymers also show an intermediate solubility behaviour, such as poly(ethylene oxide), poly(vinylpyrrolidone), those macromolecules are not ionic polymers. The solubility is significantly affected by the charge density, and accordingly by the pH, but also by the nature of the end groups born by the alkyl group R² arising from the ester pending group of each repetitive unit of formula (I).

In some embodiments, the ionic polymer may be further characterized by the mean pKa of a ternary amino group which can be present on the alkyl group R² of its ester pending group. In some embodiments, this mean pKa may be typically close to neutral pH, i.e. 6.7, a value which is close to physiological pH in most biological fluids of an animal body. Accordingly, the global ionisation level of the ionic polymer is only partial and can be estimated to 30 % at pH 7.4. This relatively low density of ionic groups present on the polymer at physiological pH is advantageous to avoid any safety issues. An additional advantage of this specific pKa is to maintain a buffer capacity of the ionic polymer if in contact with a more acidic medium such as the gastric environment in case of oral administration, but also after cell internalisation.

In some embodiments, the polymer of Formula (I) can spontaneously be converted from a cationic polymer, to a polyampholyte polymer, and eventually in a later stage to an anionic polymer. Indeed, if the substituent R¹ chemically linked on the second carbon of the vinyl repetitive unit is a hydrogen, thus corresponding to an acrylate unit, a self-catalysed hydrolysis of the ester bond occurs spontaneously and progressively when this polymer is in contact with water (Truong, N. P., Jia, Z., Burges, M., McMillan, N. A. J., & Monteiro, M. J. Self-Catalysed Degradation of Linear Cationic Poly(2-dimethylaminoethyl acrylate) in Water, Biomacromolecules, (2011), 12(5), 1876-1882). In this case if originally the corresponding alkyl group R² of the ester was capped by a ternary or quaternary amino group, the resulting hydrolysis of the ester will generate a carboxylic acid susceptible to be ionized at neutral pH. Accordingly, and in function of the proportion of acrylate repetitive units in the original poly(meth)acrylate, its global charge could turn progressively from a positive sign to a neutral or even a negative sign. Accordingly, the adjustment of the proportion of acrylate repetitive unit can be used to fine tune the evolution of charge density of the ionic polymer in function of time, allowing therefore to improve the loading of the at least one proteinaceous agent in function of their own mean charge value, but also its corresponding release rate in vivo. In some embodiments, this charge inversion of the ionic polymer could also be used in order to control and avoid cell toxicity.

Advantageously, the ionic polymer does not require any molecular separation between hydrophobic and hydrophilic polymer segments or sequences. The amphiphilic properties inherently arising from the ionic polymer enables part of the polymer to migrate to the surface of PAEC and therefore contribute to their stability in aqueous medium.

The characteristics listed above make the ionic polymer particularly suitable for interaction with proteinaceous active agents. Proteinaceous active agents have a molecular conformation including hydrogen bonds, ionic bonds, but also hydrophobic interactions. In contrast to block copolymers or multisequence or multi-segment based polymers, the multiplicity and the molecular proximity of the potential groups present in the ionic polymer offer more interacting binding sites and interact more favourably with proteinaceous agents to form a Poly Amphiphilic Electrolyte Complex (PAEC). Accordingly, higher loading of one or more proteinaceous active agents is made possible compared to Block lonomer Complexes (BIC) of the state of the art.

A further advantage is that with the ionic polymer used in the invention, the release rate of a proteinaceous active agent may be temperature dependent. In some embodiments, from 4 to 20°C no significant release of the drugs was observed, whilst when incubated at body temperature, a progressive dissociation of the PAEC was noticed with a sustained release of the drugs.

In some embodiments, the ionic polymer may have a mean pKa of from 6 to 8, for example a pKa which is around physiological pH which is about 7.4. An advantage of this embodiment is that it ameliorates a problem with PEI which is that branched PEI has a pKa value of from 8.2 to 9.9 which in combination with its molecular weight (Mw) and branching level, results in a too high charge density of positive groups which is believed to cause cytotoxicity.

In some embodiments, the ionic polymer may have alpha and omega groups respectively represented by X₁ and X₂. It is well known by a person of skill in the art that the alpha and omega groups represented by X₁ and X₂ are affected by the nature of the reaction conditions used for synthesis of the ionic polymer such as the initiator, nature of co-monomers, if present, and conditions of polymerisation termination. In some embodiments, the ionic polymer may be formed by radical polymerisation, and in particular by atom-transfer radical polymerization (ATP) in which these factors are well known according to the state of the art. In some embodiments, where a co-monomer is not present, X₁ may represent an ethyl isobutyrate moiety and X₂ may represent a hydroxyl group.

In some embodiments, R² represents a straight or branched chain alkyl group comprising from 1 to 10 carbon atoms, for example from 1 to 6 carbon atoms, e.g. two carbon atoms. In some embodiments, the optional group for R² which has a positive charge at physiological pH is a group of formula: -N(R³)₂ (III) wherein each R³ substituent may be the same or different and represents a hydrogen atom or a straight or branched chain alkyl group. In some embodiments, each R³ substituent may represent a straight or branched chain alkyl group to improve haemocompatibility. In some embodiments, the straight or branched chain alkyl group represented by R³ may have from 1 to 6 carbon atoms, for example from 1 to 4 carbon atoms; particularly R³ may represent a methyl group.

In some embodiments, the repetitive unit of formula (I) may be positively charged where the R² substituent represents a straight or branched chain alkyl group which is substituted by a group which has a positive charge, for example a protonated group of formula (III) or a group of formula -NH(R³)₂⁺ (IV). The protonated group of formula (III) may be formed when the ionic polymer is dispersed or dissolved in a physiologically compatible medium such as an isotonic saline solution or phosphate buffer saline medium (PBS). The group of formula (IV) may be formed during the preparation of the polymer, for example during a purification step in a basic solution. In some embodiments, the mean charge density may be from 1, for example from 20, e.g. from 15 to 80 %, for example to 50 %, e.g. to 30%.

In some embodiments, where the ionic polymer is positively charged, the ionic polymer may include a counter ion which is an anion, for example OH⁻; Cl⁻; HC0₃⁻; N0₃⁻; and/or H₂P0₄⁻.

By co-polymer is meant a polymer comprising two or more repetitive units where the repetitive units can be organized according to a different architecture (for example linear, cyclic, grafted and/or star), sequence (for example random, block copolymer and/or micro- sequence), and/or configuration (for example atactic, isotactic and/or syndiotactic). In some embodiments, the composition of the ionic polymer may be altered by copolymerisation of monomers bearing positive charges but also by non-ionic or/and anionic groups. In some embodiments, the length of the ionic polymer may also be from some hundred to several thousand Da, as required.

In some embodiments, the ionic polymer may be a copolymer in order to modify the charge density of the ionic polymer and to limit protein adsorption. In some embodiments, the repetitive unit of formula (I) may be copolymerised with one or more of the following repetitive units: an ethylene glycol, an acrylate, a methacrylate, optionally carrying a polyethylene oxide (PEO), for example with a mean Mw typically from 400 to 5,000, or a repetitive unit of formula (I) wherein R² is substituted by a group of formula (IV). In some embodiments, the co-monomer may be randomly distributed or the co-polymer may have a block-wise or multi-block structure.

Examples of co-polymeric structures which could be used as the ionic polymer include the following:
(a) copolymer of poly[2-(dimethylamino)ethyl methacrylate)-co-acrylic acid]: 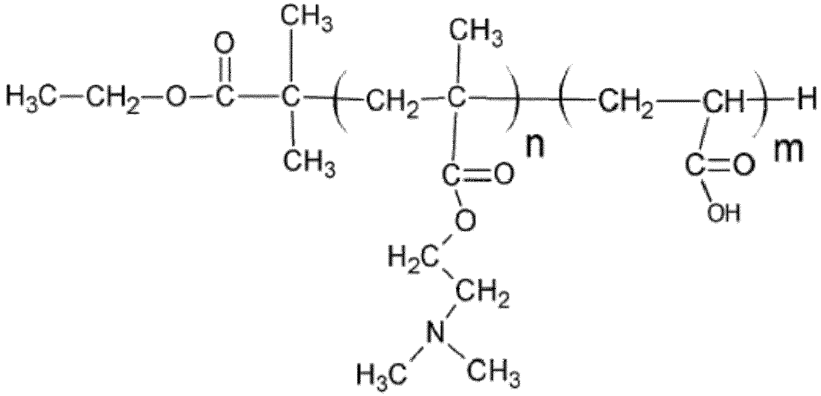
(b) copolymer of poly[2-(dimethylamino)ethyl methacrylate)-co-methacrylic acid]: 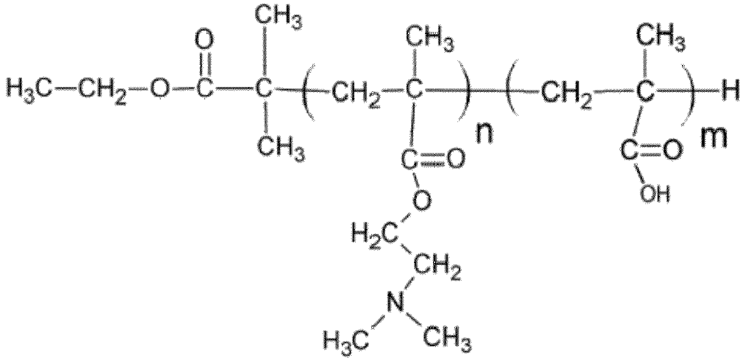
(c) copolymer of poly[2-(dimethylamino)ethyl methacrylate)-co-poly(ethylene glycol)a-methyl ether, co-acrylate] : 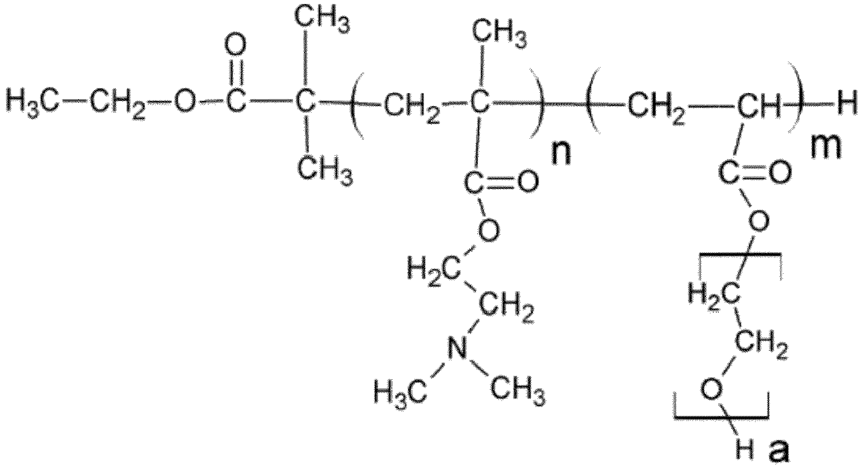
(d) copolymer of poly[2-(dimethylamino)ethyl methacrylate)-co-poly(ethylene glycol)a-methyl ether, co-methacrylate] : 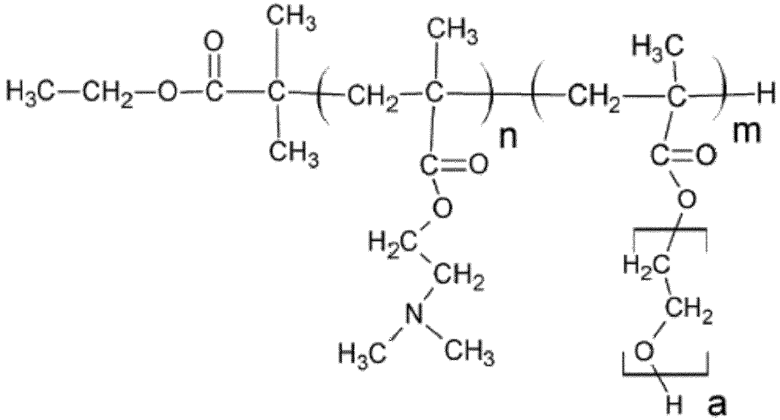
(e) copolymer of poly[2-(dimethylamino)ethyl methacrylate)-co-poly(ethylene glycol)]: 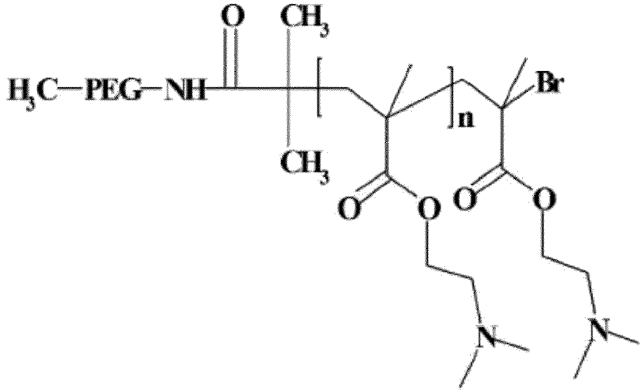
(f) copolymer of poly[2-(dimethylamino)ethyl methacrylate)-co-poly(ethylene glycol)]: 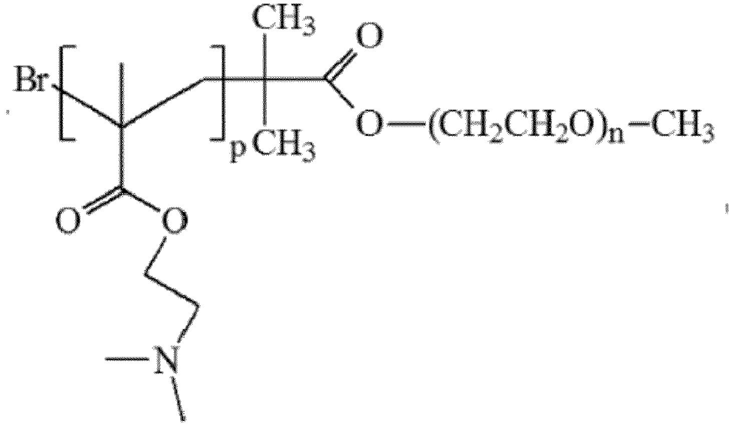
(g) terpolymer of poly[methacrylic acid-co-2-(dimethylamino)ethyl methacrylate)-co-poly(ethylene glycol)α-methyl ether, co-methacrylate]: 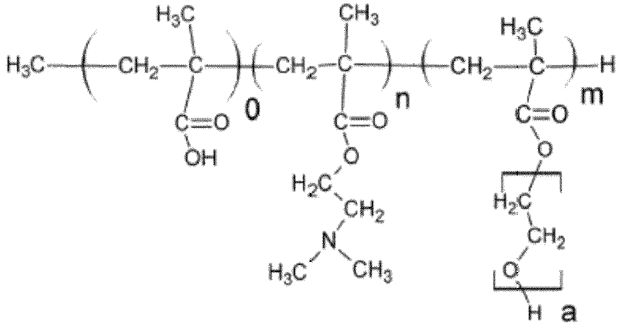
(h) terpolymer of poly[methyl methacrylate-co-2-(dimethylamino)ethyl methacrylate)-co-poly(ethylene glycol)α-methyl ether, ω-methacrylate]: 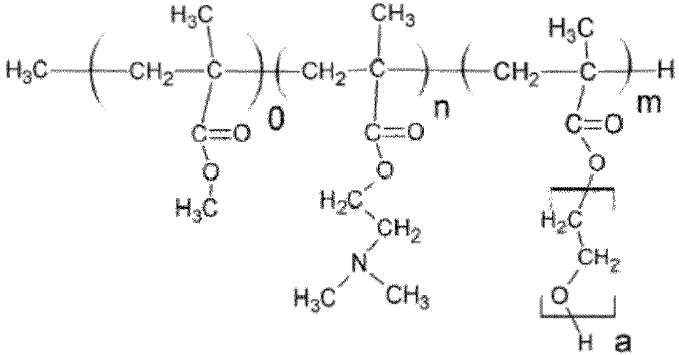
(i) terpolymer of poly[trimethylamino)ethyl methacrylate-co-2-(dimethylamino)ethyl methacrylate)-co-poly(ethylene glycol)a-methyl ether, co-methacrylate]: 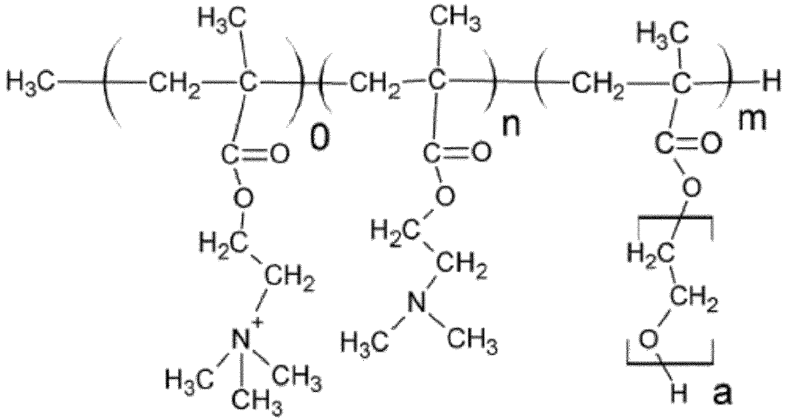
(j) copolymer of poly[trimethylamino)ethyl methacrylate-co-2-(dimethylamino)ethyl methacrylate]:

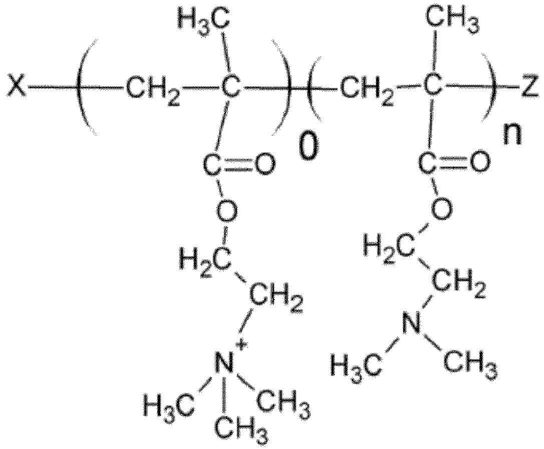

In some embodiments, the ionic polymer may be a haemo-compatible polymer. By haemo-compatible, it is meant that the ionic polymer does not elicit a substantial adverse reaction when in contact with a patient's blood or blood compartment. The criteria for haemocompatibility are detailed in ISO 10993-4 and include, for example, that there is substantially no haemolysis, no activation or inhibition of coagulation cascade, no complement activation and/or no cell activation aggregation (specifically for platelet).

Unless otherwise defined, the molecular weight (Mw or Mn) or molecular mass is expressed in Daltons (Da). In some embodiments, the ionic polymer may have a linear structure, particularly of the family of poly(meth)acrylates. In some embodiments, n represents an integer which may have a value of from 6 to 130 such that the ionic polymer of formula (II) may have a molecular weight of from 1,000, for example from 2,000, e.g. from 4,000 to 20,000, for example to 15,000, e.g. to 10,000.

The ionic polymer offers several advantages over naturally occurring polycations. In particular due to their synthetic origin, their macromolecular features can be adapted in a versatile way. For example, their mean charge density and charge distribution can be easily modified by using at least substituted and unsubstituted repetitive units whose molar proportion and distribution within the polymer of formula (II) may be random or can be tailored to control the interaction of the ionic polymer with a defined proteinaceous agent.

In some embodiments, the repetitive unit of formula (I) may comprise N,N dimethyl amino ethyl methacrylate (such that the ionic polymer of formula (II) is PolyDimethyl Amino Ethyl MethAcrylate or PDMAEMA). In some embodiments, PDMAEMA may have a mean charge density of about 30 %.

With an exponential factor of the Mark-Houwink parameters of 0.5 and 0.6, linear PDMAEMA has a relatively high expanded conformation, a macromolecular feature allowing the interaction with a proteinaceous agent. With a mean pKa of around physiological pH which is about 7.4, PDMAEMA has a relatively low mean charge density compared to PEI or poly(L)Lysine. Accordingly, its haemocompatibility and cytotoxicity is superior compared to polycations bearing too many positive charges on their backbone.

As additional advantages, the ionic polymer is easy to produce at an industrial scale and at a very low cost. Its macromolecular characteristics and purity can be easily controlled by macromolecular engineering which allows the adjustment of their composition, length, and sequence.

In some embodiments, the complex or composition according to the invention may be in dried form. In some embodiments, a dried complex or composition according to the invention may be a lyophilisate form or a spray-dried form. A skilled person would know how to prepare a suitable lyophilisate or spray-dried form of the complex or composition according to the invention. The dried (e.g. lyophilisate or spray dried) form of the complex or composition according to the invention could be reconstituted in a buffered isotonic medium just before injection.

In some embodiments, the pharmaceutical composition according to the invention may be an isotonic solution, for example a sterile isotonic solution which is optionally buffered at pH 7.4. In some embodiments, the pharmaceutically acceptable carrier may be an optionally buffered isotonic medium, for example a phosphate buffered saline medium (PBS). A suitable PBS composition is for example: composition: KH₂P0₄ 1.4 mM, Na₂HP0₄ 10 mM, NaCl 137 mM, KCl 2.7 mM and adjusted to pH 7.4. A skilled person would know how to prepare a suitable optionally buffered, sterile isotonic solution of the complex or composition according to the invention.

The pharmaceutical composition may have a concentration of the complex of from 1 µg/mL and 100 mg/mL. The concentration of the complex or of the at least one proteinaceous agent in the pharmaceutical composition according to the invention may be from 10 to 1000 times higher than the final concentration to be achieved in whole blood.

By proteinaceous is meant a peptide or a polypeptide or a protein. Many different peptides or proteins or their derivatives can be incorporated within the formulation. In some embodiments, the proteinaceous agent for use in the present invention may be an active proteinaceous agent such as a proteinaceous drug. Examples of suitable active proteinaceous agents include immunomodulators, cytokines, hormones (for example: human growth hormone or insulin), enzymes, a tissue plasminogen activator, clotting factors, colony stimulating factors, neuropeptides, recombinant soluble receptors, monoclonal antibodies, and/or erythropoietin. In some embodiments, the proteinaceous agent may be an active proteinaceous agent which has biological activity.

In some embodiments, the at least one proteinaceous agent may also include an antigenic agent for vaccination purposes. In some embodiments, where the at least one proteinaceous agent is a protein, the at least one proteinaceous agent may be in the form of a mixture with an adjuvant such as an oligonucleotide or a DNA or RNA polynucleotide, including a fragment of one or more of them.

In some embodiments, the at least one proteinaceous agent may be a synthetic proteinaceous agent (e.g. produced according to a biotechnological route), a proteinaceous agent (e.g. a proteinaceous compound) from a natural source, and/or a proteinaceous agent produced by chemical synthesis.

A surfactant can be defined as any inorganic or organic compound that is able to alter the surface properties of a given phase towards another phase. This alteration may be a partial or total migration from one phase to the other. The nature of the phase can be either liquid, solid or gaseous or a combination of both. Advantageously, a surfactant has the ability to spontaneously form molecular or supramolecular aggregates above a given concentration in the bulk phase. This threshold concentration is the critical micellar concentration (CMC). As a function of the chemical properties of the surfactants, the molecular dynamic and their stability can be strongly modified. For example, either very dynamic exchange between free and aggregated surfactants can occur, typically on a microsecond range time scale, either the mean residence time of surfactant within stable micelles can extend for days or months if stable, although not covalent, bonds are generated within the core of these micelles. Advantageously, a surfactant can also spontaneously adsorb or interact with the ionic polymer or the proteinaceous agent. This physisorption can proceed at a molecular level or supramolecular level and contributes to modify the solubility and stability features of these macromolecules in solution or in the colloidal or suspension state.

In some embodiments, a surfactant may be added to the at least one proteinaceous agent before addition of the ionic polymer. Advantages of this approach include that it may help to control the formation of PAEC and its cohesiveness, as well as its colloidal and biological stability. Different types of surfactants can be adopted. In some embodiments, the surfactant may be a cationic, anionic, non-ionic and/or zwitterionic surfactant.

According to the invention, there is also provided a process of preparing a complex comprising an ionic polymer, at least one proteinaceous agent, and a surfactant which method comprises the following steps:
(a) mixing at least one proteinaceous agent with a surfactant; and
(b) mixing the product of step (a) with an ionic polymer to form a complex.

Advantages of the process according to the invention include, in contrast to other authors who have intended to change the amphiphilicity of their ionic block copolymer by their preliminary complexation with surfactants (A.V. Kabanov, A. Eisenberg, V.A. Kabanov. Composition for delivery of biological agents and methods for the preparation thereof, US 7,169,411), that preliminary association of the surfactant with the proteinaceous agent allows the mean density of surface charge of the proteinaceous agent and their relative surface hydrophilicity to be adjusted.

In some embodiments, the surfactant may be amphiphilic, having at least one hydrophobic group and at least one hydrophilic group. In some embodiments, the at least one hydrophobic group and at least one hydrophilic group are separated enough at a molecular level in order to guarantee the difference in solubility and affinity between them. Advantages of such a differentiated amphiphilic surfactant include that It would then be able either to dissolve or to disperse in various mediums, such as aqueous and hydrophobic phases but also medium of intermediate solubility. However, their solubility will be always limited in these medium and they will spontaneously migrate and adsorb preferentially to interphases where their respective hydrophilic and hydrophobic segments can better interact. The chemical nature of the surfactants may be extensively wide, bearing either non-ionic segment(s), and/or ionic segment(s), and/or anionic group(s). In some embodiments, the molecular weight of the optional surfactant may be from 100 Da to several thousand kDa. In the latter case these macromolecules are typically made from co-polymers with block-, graft-, or dentrimetric architecture to allow a better molecular separation between hydrophilic and hydrophobic segments.

Examples of suitable surfactants accepted for pharmaceutical purpose are ethoxylated fatty alcohol, non-ionic di- or multi-block copolymers, salts of fatty acids (such as oleic acid, arachidonic acid, and/or cholesterol derivatives), salts of sulphate alkyl chains (such as sodium dodecyl sulphate or SDS). In some embodiments, the surfactant may have a HLB (Hydrophilic Lipophilic Balance scale) between 7 to 30.

Advantages of the addition of a surfactant include that:
- it may help to modify the interaction strength between the proteinaceous agent(s) and the ionic polymer;
- the presence of a surfactant can affect the physico- but also the biological stability of the complex made between the ionic polymer and the proteinaceous agent(s);
- possible preferential migration of the surfactant to the external surface of the complex can enhance the stability of the complex in physiological medium containing ions at high concentration but also large quantities of various proteins which could adsorb to the surface of the complex (or nanovehicle);
- the surfactant may advantageously be used to affect the interplay and dynamic of interaction between the proteinaceous agent and the ionic polymer; and
- by modifying the interaction between the proteinaceous agent and the ionic polymer, the surfactant can modulate their dissociation rate in function of a triggering agent/event, such as a change in local pH and the temperature.

Further advantages of the invention include that the complex and composition may be prepared in a straightforward way without requiring any expensive mechanical tools and without needing any hazardous organic solvents. In some embodiments, the preparation of the complex and the composition generally comprises physical mixing of the ionic polymer with at least one proteinaceous agent (for example, a peptide- or protein-based drug) according to a given sequence of product addition: the drug is first added and then the polymer. If the complex or composition includes a surfactant, the surfactant is advantageously added before the polymer. The pH and ionic strength of the solution are important parameters to promote the formation of the complex (PAEC). The molecular weight and mean charge density of the polymer are also important parameters, that depend on the nature of the at least one proteinaceous agent. Specifically, when dealing with the immobilization of a proteinaceous agent which is a peptide/protein (P/P) proteinaceous agent presenting an acidic isoelectric point (i.e. pl below 7.0), a cationic polymer is needed. On the contrary, if the pl of the P/P proteinaceous agent is basic (i.e. above 7.0), an anionic polymer at the pH of the formulation process is needed.

If this rule of polymer selection is applied for most of the P/P proteinaceous agents, in some cases, it is more desirable to first reverse the global charge of the P/P drug in order to promote its interaction with a functional polymer carrying charges of a nature identical to the native properties of the P/P proteinaceous agent.

As an example, a P/P proteinaceous agent characterized by a basic pl, thus presenting a global positive charge at neutral pH can first be modified with a negatively charged (or anionic) surfactant. When mixed in appropriate conditions, such a surfactant is able to reverse the global charge of the P/P proteinaceous agent and therefore will promote its interaction with a functional polycation to produce the complex or PAEC. An example of a suitable surfactant for this purpose is sodium lauryl sulfate (sodium dodecyl sulfate or SDS), or other sodium salts of fatty acids such as oleic acid or arachidonic acid. This solution can also be reversed when considering a P/P proteinaceous agent with an acidic pl. In the latter case the electrokinetic potential of the P/P proteinaceous agent may be therefore modified by the addition of an ionic surfactant such as a phosphocholine derivative such as dilauroylphosphatidylcholine.

In some embodiments, the complex according to the invention may have a size range of from about 10 nm, e.g. from 100 nm, to about 10 µm, e.g. to about 1 µm. For example, the complex may have a mean size around 50, 100, 200 or 500 nm. In some embodiments, the mean size may be measured by dynamic light scattering (DLS).

### Applications

The complex (or PAEC) can be administered through different ways including but not limited to oral, nasal, pulmonary, topical (or skin), or parenteral administration in order to enhance the diffusion of drug through mucosal barrier. Moreover, due to the minute size of these carriers and their haemocompatibility, they can be also customized in order to be injectable parenterally, subcutaneously, intramuscularly or intravenously in view to facilitate their access to their pharmacological sites, including for the purpose to cross the Blood-Brain-Barrier (BBB). More specifically it should be stressed that the administration of these nanodispersions by nasal administration promotes their resorption in the systemic circulation and can also give direct access to the central nervous system (CNS), therefore avoiding the difficult passage of the BBB. The haemocompatibility and cell biocompatibility of the ionic polymer has been previously reported in the literature.

In some embodiments, depending on the mode of administration, composition according to the invention and/or the complex (or PAEC) according to the invention may be delivered in the form of: a suspensions (for example in an isotonic medium buffered at neutral pH), an aerosol (e.g. for nasal, pulmonary, or topical/skin application), a viscous liquid (e.g. for intramuscular or other parenteral routes in order to limit their diffusion and increase their local therapeutic action at the site of injection), a liquid able to generate a gel once injected within the body, or a gastro-resistant capsule. In some embodiments, a gastro-resistant capsule may have a pH-sensitive polymer coating which is selected to dissolve after passage in the small intestine to release the complex (or nanoparticles). In some embodiments, the complex (or PAEC) may be immobilized in an alginate microcapsule to protect the complex from a gastric environment.

In some embodiments, the complex (or PAEC) and/or composition may be for immunological use. In some embodiments, the complex and/or composition may be a vaccine. In some embodiments, an immunological complex and/or composition may comprise an immunological proteinaceous agent (such as an amino acid, peptide, or protein) which generates an immune response (such as an antigen) in a human or animal body. In some embodiments, an immunological complex and/or composition may comprise an immunoadjuvant to enhance the immunoreactivity of the proteinaceous agent (such as an antigen) upon its contact with an immunocompetent cell.

In some embodiments, the complex may consist essentially (or may consist) of at least one proteinaceous agent, an ionic polymer comprising a repetitive unit of formula (I), and optionally a surfactant, and optionally an alginate.

The invention will now be illustrated with reference to the following Figures of the accompanying drawings which are not intended to limit the scope of the claimed invention:
**Figure 1** illustrates the results of Example 1 and shows graphs which illustrate the evolution of the mean light scattering Intensity (Id cpm) and mean diameter of the PAEC;
**Figure 2** illustrates the results of Example 1 and shows a comparison of an electrophoretic profile of free ovalbumin versus ovalbumin associated with ionic polymers, either OB006 (PDMAEMA with a Mw of 10 kDa) and DA002 (PDMAEMA with a Mw of 90 kDa);
**Figure 3** illustrates the results of Example 2 and shows graphs which illustrate the evolution of the mean light scattering Intensity (Id cpm) and mean diameter of the PAEC loaded with human insulin;
**Figure 4** illustrates the results of Example 2 and shows CD spectra analysis of free insulin or associated with an ionic polymer;
**Figure 5** illustrates the results of Example 2 and shows TEM images (20,000x magnification where the horizontal line on each image represents a distance of 1µm at that magnification) of PAEC loaded with human insulin with and without PEG;
**Figure 6** illustrates the results of Example 4 and shows graphs illustrating the kinetics of formation of quaternary PAEC made from CpG, P24, SDS and an ionic polymer (DA002) controlling the mean light scattering intensity (Id) and the mean size of the PAEC suspension by dynamic light scattering (DLS);
**Figure 7** illustrates the results of Example 4 and shows a graph illustrating the stability of quaternary PAEC made from CpG, P24, SDS and an ionic polymer (DA002) made with P24 loading ranging from 50 to 250 µg/mL;
**Figure 8** illustrates the results of Example 4 and shows a comparison of the mean diameter measured by DLS (shown on y-axis by units of nanometres) of 6 different batches of PAEC loaded with respective concentrations of P24/CpG/SDS/ionic polymer DA002 were 200 / 80 / 200 / 350 µg/mL. The batch number 5 has also been submitted to 3 freeze-thawing cycles;
**Figure 9** illustrates the results of Example 4 and shows a comparison of the electrophoretic profile of free P24 with P24 associated with cationic polymers, either OB006 or DA002;
**Figure 10** illustrates the results of Example 4 and shows a comparison of the electrophoretic mobility of PAEC loaded with respective concentrations (µg/mL) of P24/CpG/SDS/ionic polymer DA002 in comparison to latex (track 13);
**Figures 11A and 11B** show TEM micrography images (5.000x magnification where the horizontal line on each image represents a distance of 500nm at that magnification) which illustrate the results of Example 4;
**Figure 12** shows a bar chart which illustrates the results of Example 5 by mean light scattering intensity (Id) measured by DLS of PAECs;
**Figure 13** shows a bar chart which illustrates the results of Example 4 which are an analysis of the medium term stability of the PAEC loaded with CpG and P24 in a PBS medium at temperatures of 4, 25 or 37°C. The evolution with time of the mean light scattering Intensity has been analyzed up to 90 days;
**Figure 14** shows TEM micrography images (5.000 x) which illustrate the results of Example 6 which are of alginate microbeads loaded with binary PAEC (a) and Alginate microbeads loaded with ternary PAEC stabilized with SDS (b); and
**Figure 15** shows a bar chart which illustrates the results of Example 7 which are the mean size (measured by mean diameter (nm) on the y-axis) of three batches of PAEC.

The invention will now be illustrated in the following Examples which are not intended to limit the scope of the claimed invention.

### EXAMPLES

Our formulation was tested with several proteinaceous drugs, namely human insulin, ovalbumin or a combination of an antigenic protein and an immunoadjuvant such as an oligonucleotide. The formation and stability of these PAEC was analysed with the following methods:
i. Dynamic Light Scattering (DLS) giving the mean light scattering Intensity (Id) and autocorrelation function evolution, indicates the formation of the complex and its size as well as stability of PAEC (ionic strength, time, mechanical solicitation)
ii. Electrophoretic Light Scattering (Coulter Delsa) giving electrophoretic mobility
iii. Electrophoresis conducted in non-denaturating conditions to evaluate drug loading after separation of PAEC from the free peptide/protein
iv. HPLC to determine free surfactant concentration
v. TEM microscopy to analyse the morphology of the PAEC
vi. In vitro release kinetics of the proteinaceous drugs
vii. Circular dichroism to control the conformation and hence stability of the proteinaceous drug

### Example 1. Preparation of binary polyelectrolyte complexes with ovalbumin

PAEC is prepared by physical mixing of aqueous solutions at room temperature. The aqueous solutions are previously filtrated on sterile 0.2 µm filters and the whole formulation procedure is realized within a laminar flow. The proteinaceous drug is ovalbumin, a protein of chickens which consists of 385 amino acids with a relative molecular mass of 42.7 kDa and with a serpin-like structure in a native status and an isolectric point of 4.5 (P.E. Stein, A.G.W. Leslie, J.T. Finch,; R.W. Carrell, Crystal structure of uncleaved ovalbumin at 1,95 Å resolution". Journal of Molecular Biology 1991, 221 (3): 941-959). The ionic polymer is poly(2-dimethylamino)ethyl methacrylate (PDMAEMA), with a molecular weight from 7 kDa to 91 kDa) dissolved in a sodium phosphate buffer medium equilibrated at a pH of about 7.4. Its concentration is ranging between 0.1 to at least 10 mg/mL, depending on the final drug loading, typically 10 to 50 wt %. Ovalbumin is dissolved carefully in the same buffer solution.

The mixing of the ionic polymer and ovalbumin solutions is performed in a polypropylene tube. The protein solution is typically first added in the appropriate vessel and a given volume of ionic polymer solution is quickly added in one time with a suitable injection device, directly within the solution and not against the wall of the vessel. The volume ratio of protein solution to the ionic polymer solution ranges between 1:1 to at least 100:1 and is adjusted in order to afford a rapid and homogeneous distribution of the ionic polymer within the protein solution. Ionic polymer volume is for example 1/10 of the total volume of PAEC solution.

PAEC formation between ovalbumin and the ionic polymer has been verified by DLS, monitoring both the increase in mean light scattering intensity and the appearance of an autocorrelation curve.

The size distribution in intensity of the PAEC's have been calculated after deconvolution of the autocorrelation curves and disclosed a mean radius in Intensity of PAEC in the nanosize range.

| **Table 1A** | | **Table 1B** | |
|---|---|---|---|
| Mw | Radius at 50% (nm) | lonisation % | Radius at 50% (nm) |
| 7000 | 236 +/- 22 | 30 | 248 +/-13 |
| 13000 | 248 +/- 13 | 47 | 353 +/- 7 |
| 26400 | 453 +/- 5 | 60 | 210 +/- 8 |
| 49100 | 5171 +/- 225 | 80 | 222 +/- 6 |
| 91400 | 2852 +/- 120 | | |

Table 1A shows the evolution of the mean radius (percentile 50) of PAECs loaded with ovalbumin as a function of the Mw of ionic polymer keeping constant the ionisation percentage to 30 %. Table 1B shows the percentage of ionic groups in relation to the total number of repetitive units in the ionic polymer of PAECs loaded with ovalbumin where the ionic polymer has a Mw of 13 kDa.

Different molecular weights (Mw) and charge density of ionic polymer were tested keeping all experimental parameters constant. The mean size of the obtained PAECs (Table 1A and B) by DLS highlights that low Mw of ionic polymer (i.e. < 20 kDa) are giving rise to smaller polyelectrolyte complexes while the variation in the number of ionizable groups per macromolecule chain has less significant impact on the mean size of the PAECs loaded with ovalbumin.

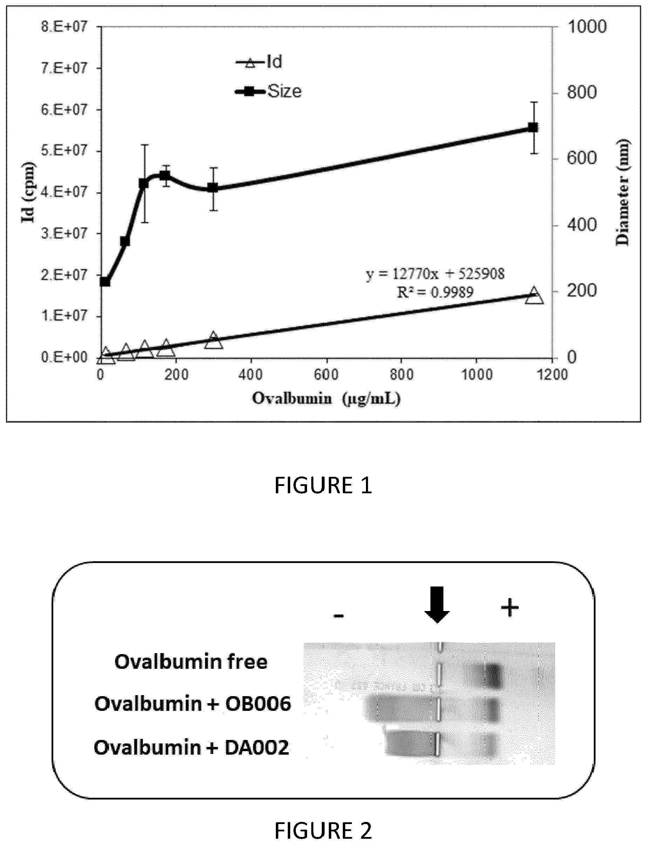

Furthermore, PAECs were prepared with ovalbumin solutions of concentration ranging from 15 µg/mL up to 1150 µg/mL, while keeping constant the weight ratio between the protein and the ionic polymer (1/2). Figure 1 shows the evolution of the mean light scattering Intensity (Id cpm) and mean diameter of the PAEC loaded with ovalbumin in function of the concentration of ovalbumin used in the formulation. As outlined on Figure 1, a linear relationship was observed between the mean light scattering intensity of the PAEC nanodispersions loaded with ovalbumin and its concentration. This linear increase in DLS signal is supporting the efficiency of interaction between the proteinaceous drug and the ionic polymer in an extended range of concentration. The mean size of the PAECs remains in the colloidal range.

The efficiency of drug immobilization and drug loading within PAECs was assessed after separation of the free protein from PAEC with a suitable ultrafiltration device with a membrane cutoff selected to retain the PAEC. The concentration of free ovalbumin in the ultrafiltrate, measured using BCA as protein bioassay, has been estimated to 42%, therefore corresponding to a loading effectiveness of 58%. These two percentages are given with respect to the total amount of ovalbumin added originally in the formulation. Besides, a gel electrophoresis in non-denaturation conditions was performed, with deposition of the samples in the center of the gel disposed horizontally. Adopting this geometry, the PAEC and the free proteins could migrate in opposite direction as a function of their own electrokinetics potential sign and intensity.

Figure 2 shows a comparison of the electrophoretic profile of free ovalbumin versus ovalbumin associated with ionic polymers, which are either OB006 (PDMAEMA with a Mw of 10 kDa) or DA002 (PDMAEMA with a Mw of 90 kDa). The electrophoretic profile used gel electrophoresis performed in non- denaturation conditions. The signs + and - which are arranged to the right and left of the vertical arrow indicate the polarity of the electric field, which was applied, while the arrow indicates the location of the sample deposition. After separation, the gel was stained using a silver technique. Figure 2 highlights the difference in electrophoretic direction. Indeed, when associated to one of the ionic polymers, ovalbumin is migrating in direction of the cathode, while free ovalbumin, used as control, moved in direction of the anode. Knowing that the samples and the gels have been equilibrated in a medium buffered at pH 7.4 before proceeding to the electrophoresis, these results are demonstrating the reversibility of the electrokinetic potential of ovalbumin after complexation with an ionic polymer of formula (I).

### Example 2. Preparation of binary polyelectrolyte complex with human insulin

Human insulin is a small globular protein having a Mw of 5808 Da made of 52 amino acid residues distributed in two polypeptide chains, chain A (21 residues) and B (31 residues) which are linked by disulfide bonds. Its isoelectric point is 5.3. Despite its relatively small size, insulin is injected parenterally to diabetic patients on a daily basis in order to reduce the risk linked to the development of microvascular and macrovascular complications, which are one of the main cause of morbidity and mortality associated with this disease. Insulin represents therefore an important proteinaceous drug which could benefit from a nanocarrier allowing to cross biological mucosa and improve the bioavailability.

Poly (2-dimethylamino) ethyl methacrylate is dissolved in a buffer medium as in Example 1. Insulin dissolution requires first dissolution in an acidic medium, such as acetic acid before proceeding to the progressive neutralisation of the protein solution to achieve a neutral pH. Important precautions should be taken to standardize the conditions of insulin dissolution to avoid any molecular aggregates or either micro- or macroscopic aggregates. Accordingly, the dissolution of this protein should be realized using a device which limits shearing and prevent the introduction of air and foam formation during mixing. In a typical dissolution protocol, insulin is prepared at a final concentration of from 0.1 to at least 10 mg/mL by dissolving the lyophilisated powder in acetic acid solution (0.6M). Placed under rotation, the solution is achieved within a time scale of 10 min at room temperature. Neutralisation of this solution is performed by the progressive addition of a strong base solution, such as NaOH (M) and under a careful control of the pH. When achieving at least pH 6.5 the insulin solution should be transparent, but NaOH should still be added in order to reach pH 7.40. Once arrived at the expected concentration, it is also important to future equilibrate the insulin solution in the same buffer which will be used afterwards to prepare the polyelectrolyte complexes. Final insulin solution should be sterilized by sterile filtration conducted within a laminar flow and can stored for future use at -20°C.

Once dissolved the insulin and the ionic polymers solutions are mixed within a standard recipe according to the same typical procedure disclosed in example 1. At the laboratory scale, this mixing is performed within a polypropylene tube or any other container of a volume adapted in function of the total volume of PAEC suspension requested. PAEC formation between insulin and the ionic polymer has been verified by DLS monitoring both the increase in mean light scattering intensity and the appearance of an autocorrelation curve. From a noisy autocorrelation curve observed in the presence only of, either of the ionic polymer solution or of the insulin solution, a clear exponential curve is observed quickly after mixing these two macromolecules.

The size distribution in intensity of the PAEC's have been calculated after deconvolution of the autocorrelation curves and disclosed a mean radius in intensity of PAEC in the nanosize range (Table 2).

In order to identify the optimal macromolecular features of the ionic polymer allowing to control its association with human insulin, we have assessed the influence of their molecular weight (Mw from 13 kDa to 91.4 kDa) and their charge density keeping constant the concentration of the protein and of the polycation (15 and 30 µg/mL respectively).

Interestingly, compared to results observed with PAECs loaded with ovalbumin, the Mw and the total number of ionizable groups per macromolecule affects also the mean size of PAECs containing an ionic polymer and insulin but in a different way. Indeed:
- a significant evolution of the DLS results is observed between analysis realized 5 and 30 min after mixing the ionic polymer and insulin. These changes of PAEC aggregation with time, not observed with ovalbumin, strongly suggests that the kinetics of the formation of these polyelectrolyte complexes is slower with human insulin;
- a decrease of the mean size of the PAEC is observed with a higher molecular weight ionic polymer, a result which contrasts to the opposite evolution noticed with ovalbumin loaded with the same polymers;
- the dependence of PAEC formation with the charge density of the ionic polymer is also different compared to results acquired with ovalbumin, the mean size of the nanodispersions decreasing by raising the charge density of the polycations.

| **Table 2A** | | | **Table 2B** | | |
|---|---|---|---|---|---|
| | T 5' | T 30' | | T 5' | T 30' |
| Mw | Radius at %50 (nm) | % 50 (nm) | % ionisation | Radius at %50 (nm) | Radius at %50 (nm) |
| 13000 | 664 +/- 20 | 5752 +/- 345 | 30 | 664 +/- 15 | 5752 +/- 345 |
| 26400 | 1527 +/- 50 | 730 +/- 34 | 47 | 2753 +/- 205 | 350 +/- 120 |
| 49100 | 731 +/- 4 | 350 +/- 23 | 60 | 847 +/- 22 | 318 +/- 35 |
| 91400 | 300 +/- 35 | 146 +/- 35 | 80 | 847 +/- 25 | 200 +/- 22 |

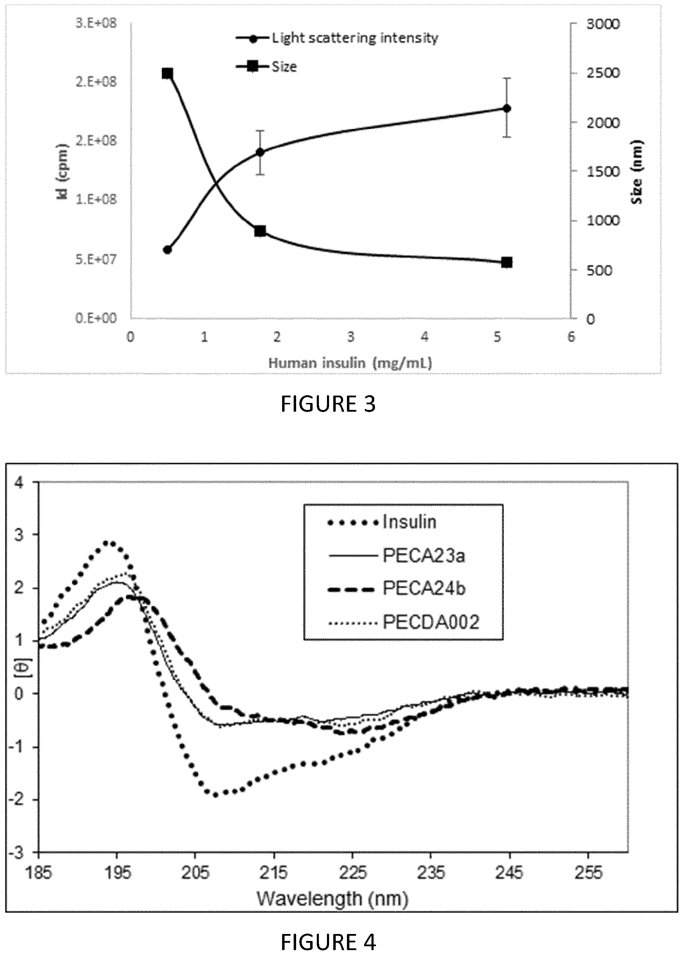

Table 2A shows the evolution of the mean radius (percentile 50) of PAECs loaded with human insulin in function of the Mw of ionic polymer keeping constant the ionisation % to 30 %. Table 2B shows the evolution of the mean radius (percentile 50) of PAECs loaded with human insulin in function of the % of ionic groups to the total number of repetitive units in the ionic polymer adopting a Mw of the ionic polymer of 13 kDa. Figure 3 shows the evolution of the mean light scattering Intensity (Id cpm) and mean diameter of the PAEC loaded with human insulin in function of the concentration of human insulin used in the formulation, keeping the weight ratio between the protein and the ionic polymer constant at 1:2.

PAECs were loaded with human insulin in a concentration of from 500 µg/mL up to 5000 µg/mL, while keeping the weight ratio between the protein and the ionic polymer constant at 1:2. Figure 3 shows graphs which illustrate the evolution of the mean light scattering Intensity (Id cpm) and mean diameter of the PAECs in function of the concentration of human insulin used in the formulation. The left hand y-axis represents a scale for Id in cpm, the right hand y-axis represents a scale for diameter in nanometres, and the x-axis represents a scale for the human insulin concentration in mg per millilitre. As outlined on Figure 3, an increase in mean light scattering intensity of the PAEC nanodispersions is observed this relationship is however not linear as noticed for ovalbumin. But surprisingly enough this increase of the ionic polymer and protein concentration is linked to a 5 times reduction in the mean size of the nanodispersions. This is advantageous because a decrease in size of PAEC is a benefit to enhance their diffusion within the body and to avoid their capture by immunological systems. This result is surprising because when the concentration of the ionic polymer and of the proteinaceous agent is increased, the probability of particle collision and therefore the risk of aggregation is increased.

The efficiency of drug immobilization and drug loading within PAECs has been assessed after separation of the free protein from PAEC adopting a suitable ultrafiltration device with a membrane cutoff selected to retain the PAEC. The concentration of free human insulin in the ultrafiltrate, measured using BCA as protein bioassay, has been estimated to 73 % when combined with an ionic polymer in a protein to polymer wt ratio of 1:2. This percentage is given with respect to the total amount of human insulin added originally in the formulation.

The comparison of the electrophoretic profile of free human insulin versus insulin associated with PDMAEMA demonstrated the reversion of the electrokinetic potential of human insulin upon association to this ionic polymer.

In view to detect some further possible changes in the conformation of the human insulin after its association with the ionic polymer, we have compared its circular dichroism spectra (CD) acquired either on the free form of this protein, or either after its association with 3 ionic polymers. From these CD spectra we have calculated the % of helix in human insulin overtime of storage at 4°C on 12-day period. This data, illustrated in Figure 4 and set out in Table 3, highlight that CD spectra of free insulin changes significantly on one-week period whilst after complexation with the polymer A23, the polypeptide gives a rather similar signal. This CD spectra comparison therefore supports that the association of human insulin with ionic polymer provides a stabilisation of the secondary structure of this protein. In particular, Figure 4 shows CD spectra analysis of either free insulin or insulin associated to an ionic polymer: A23 (PDMAEMA homopolymer with 26.4kDa); B24 (PDMAEMA homopolymer with 14kDa with a mean ionisation of 60% after quaternization) or DA002 (PDMAEMA homopolymer with 90kDa). The CD spectra were taken one day after PAEC preparation.

Table 3 shows the percentage helix content in human insulin calculated from the ratio ([θ]₂₂₂/ [θ]₂₂₃) determined from the CD spectra of either free insulin or of insulin after its association with one of these ionic polymers, A23, B24 or DA002. The CD analysis was carried out after storing the PAEC for 1, 6 or 12 days at 4°C.

**TABLE 3**

| | Day 1 | Day 6 | Day 12 |
|---|---|---|---|
| Human Insulin | 14.6 | 37.4 | 36.9 |
| PECA23p-2A1 | 22.6 | 16 | 19.8 |
| PECB24p-2A1 | 31.3 | 23.3 | 16.9 |
| PECDA002-2A1 | 26 | 16.6 | 21.1 |

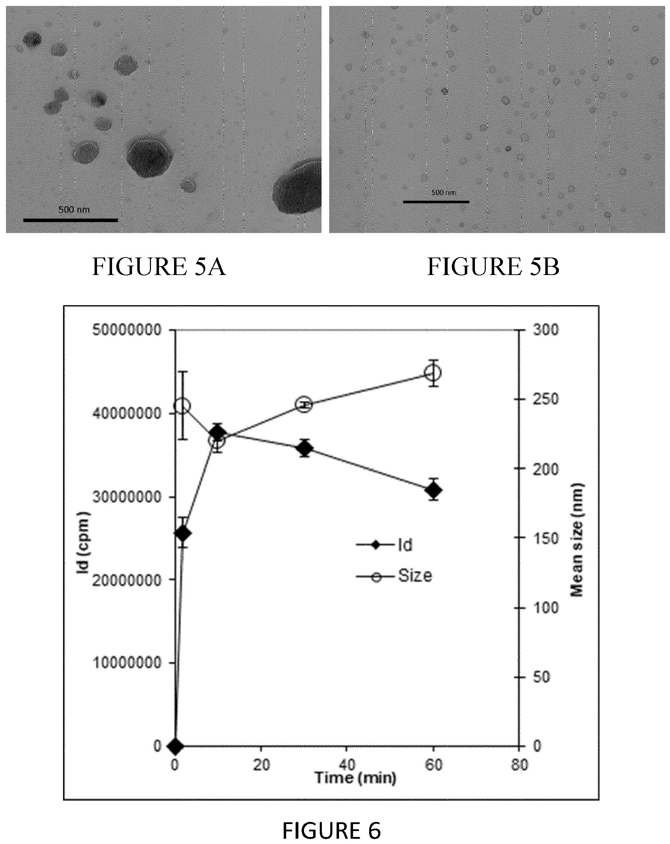

The morphology of the PAECs loaded with human insulin was also examined by transmission electron microscopy (TEM) (Figure 5). The images show the nano-range size of these particles as first observed by DLS. More homogeneous and small nanoparticles were produced when the ionic polymer was bearing a poly(ethylene oxide) sequence according to formulae where PEO and PDMAEMA have Mw of 500 and 7000 respectively (Figure 5B). In particular, Figure 5A shows TEM images (at 20x magnification) of PAECs loaded with human insulin with PDAEMA without poly(ethylene oxide). Figure 5B shows TEM images (at 20 000x magnification) of PAECs loaded with human insulin with an ionic polymer was bearing a poly(ethylene oxide) sequence according to formulae where PEO and PDMAEMA have Mw of 500 and 7000 respectively.

### Example 3. Preparation of ternary polyelectrolyte complexes with human insulin and a surfactant

Ternary polyelectrolyte complexes made from human insulin, an ionic polymer referred to as DA002 and surfactant, namely sodium dodecyl sulphate (SDS) were prepared. To a sodium phosphate buffer medium, 333 µL of human insulin solution (6 mg/mL) and 125 µL or 375 µL of SDS (20 mg/ml) were added. Five minutes after addition of SDS, the formation of PAEC was formed by the rapid addition of 1000 µL of the ionic polymer DA002 (10 mg/mL).

The influence of the molecular weight was assessed (Mw from 10 kDa to 90 kDa) keeping constant the concentration of the protein and of the polymer (2 and 4 mg/mL respectively) and a SDS concentration of 1mg/mL. The results are shown in Table 4. Interestingly compared to results observed with binary PAECs loaded with human insulin without surfactant, the Mw is affecting the mean size of PAECs in a similar way. Indeed, a decrease of the mean size of the PAEC is observed adopting higher molecular weight ionic polymer, a result which contrasts to the opposite evolution noticed with ovalbumin loaded with the same polymer. Only a slight increase in mean size is noticed 1 day after PAEC preparation and storage at room temperature.

**Table 4** Evolution of the mean radius (percentile 50) of ternary PAEC made from human insulin, SDS and ionic polymer of Mw ranging from 10000 to 90000. DLS mean size have been measured 1h and 1 day after PAEC preparation and storage at room temperature.

**TABLE 4**

| | T 1h | T 24h |
|---|---|---|
| Mw PDMAEMA | Radius at %50 (nm) | Radius at %50 (nm) |
| 10000 | 4872 +/- 2850 | 94863 +/- 11082 |
| 20000 | 206 +/- 8 | 260 +/- 2 |
| 40000 | 231 +/- 8 | 260 +/- 26 |
| 90000 | 266 +/- 4 | 315 +/- 14 |

### Example 4. Preparation of quaternary polyelectrolyte complexes with vaccine protein (P24), an oligonucleotide adjuvant (CpG) and a surfactant (SDS)

P24, a structural protein of HIV viral capside was selected as model biopharmaceutical active for vaccine delivery purpose. This recombinant protein antigenic protein has a Mw around 20 KDa.

CpG, a short single-stranded synth etic DNA molecule, is well-known to boost the generation of humoral and cellular vaccine-specific immune responses, has been co-immobilized with P24 within PAEC made of one of the ionic polymers corresponding to formula I, for example an homopolymer made from DMAEMA with a Mw of 90 kDa.

P24 was provided as a 1 mg/mL stock solution and stored at -70°C. After being thawed for at least 15 min at room temperature, this protein was diluted in a sodium phosphate buffer. CpG, under a sodium form, has also been dissolved at 1 mg/mL in a sodium phosphate buffer.

A surfactant solution, such as SDS, is prepared under a stock solution of 1 mg/mL in sodium phosphate buffer solution. This solution is further diluted in the buffer medium in order to adapt its concentration to the required drug loading.

Once equilibrated at room temperature, the P24 protein solution and the CpG solution are first mixed with the surfactant solution. Typically, CpG and P24 are mixed in a 1/1 weight ratio, while the ionic polymer is present in a 2 times excess to P24. The final concentration of the surfactant is fixed as a function of the total amount of protein P24 immobilized within the PAEC. This solution is equilibrated for some minutes at 25°C. The ionic polymer is then added.

PAEC formation between P24, CpG, a surfactant and the ionic polymer has been first verified by DLS. Figure 6 shows the kinetics of formation of quaternary PAEC made from CpG, P24, SDS and an ionic polymer (DA002) controlling the mean light scattering intensity (Id) and the mean size of the PAEC suspension by DLS. The final concentration of P24 was fixed at 250 µg/mL. The left hand y-axis shows Id (cpm) values for the line with diamond shaped data points and the right hand y-axis shows mean size (nm) values for the line with circular shaped data points.

As can be seen from Figure 6, the kinetics of formation is rapid with a maximum in mean light scattering intensity (Id) already noticed 10 min after addition of the ionic polymer. With a P24 concentration of 250 µg/mL, the mean size of the PAEC suspension ranges between 250- 270 nm. Figure 6 shows graphs illustrating the kinetics of formation of quaternary PAEC made from CpG, P24, SDS and an ionic polymer (DA002) controlling the mean light scattering intensity (Id) and the mean size of the PAEC suspension by dynamic light scattering (DLS). The final concentration of P24 was fixed at 250 µg/mL.

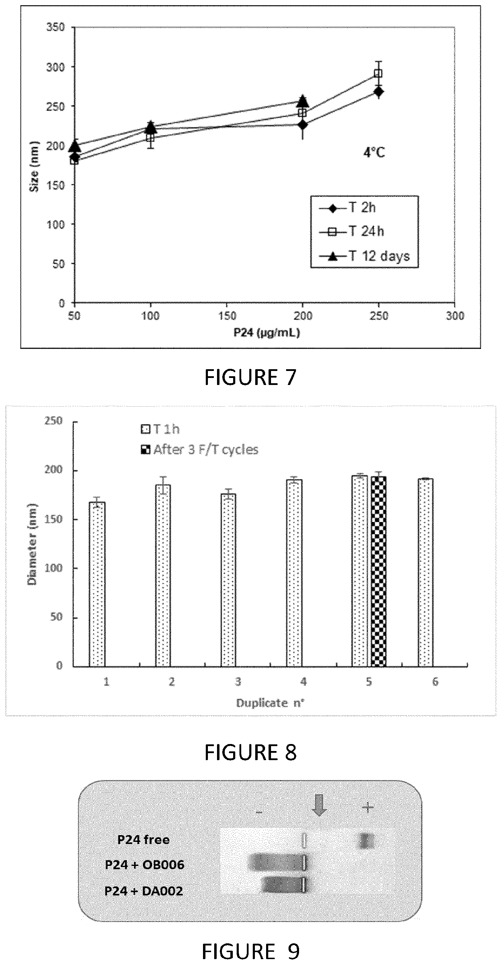

Figure 7 shows the stability of quaternary PAEC made from CpG, P24, SDS and an ionic polymer (DA002) made with a P24 loading ranging from 50 to 250 µg/mL and stored at 4°C. The stability of the nanodispersions was monitored by DLS, measuring the mean size of the PAEC. Figure 7 shows a graph illustrating the stability of quaternary PAEC made from CpG, P24, SDS and an ionic polymer (DA002) made with P24 loading ranging from 50 to 250 µg/mL (shown on the x-axis) and stored at 4°C. The stability of the nanodispersions was monitored by DLS, measuring the mean size of the PAEC.

When stored at 4°C, the PAEC remains stable over a period of at least 12 days considering P24 loading in a range of at least 50 to 250 µg/mL. Surprisingly enough, when raising the concentration of surfactant, up to a critical concentration of 200 µg/ml, the PAEC nanodispersions are homogeneous and stable. Above this concentration, heterogeneous and instable dispersions were noticed. These qualitative observations have been confirmed by DLS with an increase in mean light scattering intensity and a drastic increase in mean size of the PAEC (Figure 7).

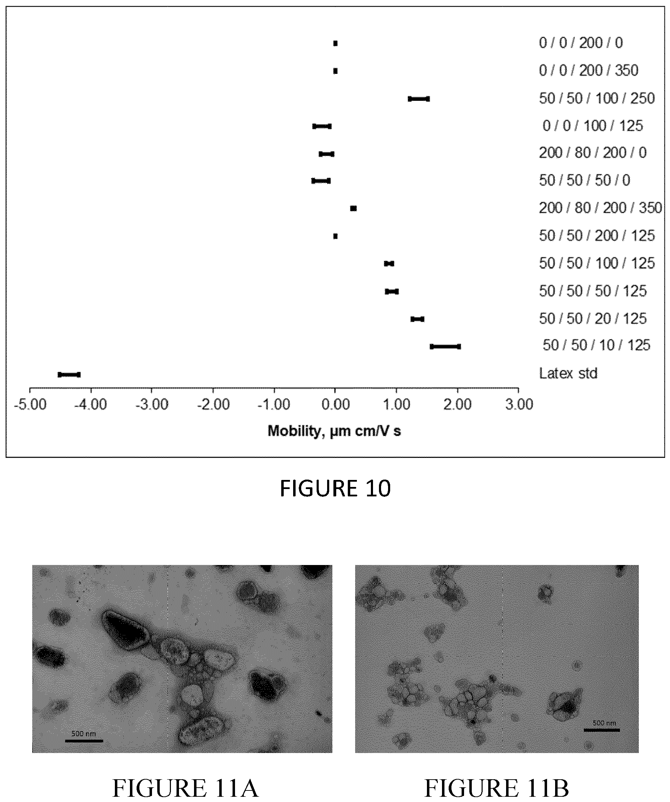

The comparison of the electrophoretic profile of free P24 associated with ionic polymers, either OB006 (PDMAEMA 10 kDa) or DA002 (PDMAEMA 90 kDa), clearly highlights a difference in electrophoretic direction. Indeed, as disclosed on Figures 9 and 10, when associated to these ionic polymers, P24 is migrating in direction of the cathode, while this protein under a free form is moving in direction of the anode. Knowing that the samples and the gels have been equilibrated in a medium buffered at pH 7.4 before proceeding to the electrophoresis, these results are demonstrating the reversion of the electrokinetic potential of P24 when associated to these ionic polymers.

Figure 8 shows the mean DLS diameter (measured on the y-axis in units of nm) of 6 batches of PAEC measured one hour after formation, one of them being submitted to 3 repetitive freeze-thaw cycle. The mean size of this batch 6 has not been affected, highlighting the stability of PAEC against this physico-chemical stress. The respective concentrations of P24/CpG/SDS/ionic polymer DA002 were 200 / 80 / 200 / 350 µg/mL. The batch number 5 has also been submitted to 3 freeze-thawing cycles.

Figure 9 shows a comparison of the electrophoretic profile of free P24 with P24 associated with cationic polymers, either OB006 or DA002, using gel electrophoresis in non-denaturation conditions. The signs + and - indicate the polarity of the electric field which has been applied, while the arrows refer to the place for sample deposition. After separation, the gel was stained using a silver technique.

Figure 10 shows a comparison of the electrophoretic mobility of PAEC loaded with respective concentrations of P24/CpG/SDS/ionic polymer DA002. Their respective concentrations in µg/mL are as follows: 0 (P24)/0 (CpG)/200 (SDS)/0 (ionic polymer) (track 1), 0/0/200/350 (track 2), 50/50/100/250 (track 3), 0/0/100/125 (track 4),. 200/80/200/0 (track 5), 50/50/50/0 (track 6), 200/80/200/350 (track 7), 50/50/200/125 (track 8), 50/50/100/125 (track 9), 50/50/50/125 (track 10), 50/50/20/125 (track 11), and 50/50/10/125 (track 12).

The electrophoretic mobility of the PAEC was analysed using a Coulter Delsa 440SX equipment, working under a constant voltage mode (10 volts) at a temperature of 25°C. The PAEC samples were first equilibrated in a phosphate buffer medium before carrying out the analysis. The evolution of the mean electrophoretic mobility of PAEC prepared according to their compositions, i.e. playing on the SDS concentration, but also on the weight ratio between P24, CpG, SDS and ionic polymers, is shown on Figure 10. This physico-chemical analysis of the surface properties of the PAEC samples allows the following findings to be drawn:
- whatever the composition or the formulation methods, the PAEC always are positively charged. This unexpected observation highlights the predominant contribution of the polycation on the electrokinetic potential of the PAEC and accordingly to its preferential migration to the PAEC surface. Accordingly, we can anticipate that the most significant part of the protein P24, of the oligonucleotide and of SDS have been associated to the ionic polymer and are mostly internalized within the core of the PAEC. Although the % in weight of the ionic polymer is typically greater than the other components of the formulation for all formulations assessed, its preferential migration to the surface of the PAEC is not straightforward. Indeed, both the protein and the surfactant or their combination, could be preferentially concentrated to the surface of PAEC keeping into consideration their well-known surface activity.
- a significant decrease in electrophoretic mobility of the PAEC is observed when raising their SDS content, but without reversing the direction of the PAEC mobility in the electrical field.
- the control made from a mixture of SDS and the ionic polymer 0/0/10/12.5 is supporting the existence of ionic complexation between this anionic surfactant and this synthetic polymer.

The fraction of SDS under a free form, i.e. not associated to the PAECs, was determined on the following composition: P24 / CpG /SDS/DA002: 200 / 80 / 200 / 350 µg/mL. This analysis was done after separation of the PAECs from free SDS, using ultrafiltration. The free SDS concentration in the permeate fraction was then determined by HPLC using an AZorbax 80A Extend-C18 column. 3.0 x 150 mm, 3.5 µm from Agilent and an Evaporative Light Scattering Detector (ELSD) Alltech^{®} Model 3300.

The HPLC analysis indicates that this purification procedure allows to retain more than 99 % of the PAEC (190 ± 2 nm in diameter) on the filter, that less than 2.5 % of the total amount of the surfactant is free, thus not associated to the polymeric nanostructures retained on the filter and that free surfactant is not adsorbed on the ultrafiltration membrane. According to this analysis, the maximum concentration of free SDS in this PAEC formulation is equal or below 5 µg/mL (Table 5).

**TABLE 5**

| | Particle recovery (Id %) * | SDS recovery (wt %) ** |
|---|---|---|
| PAEC made from p24/CpG/ SDS / DA002 | 0.06 ± 0.01 | < 2.5 |
| Free SDS | 0.78 ± 0.07 | 100.0 |
| Mixture of DA002 + SDS | 4.79 ± 1.08 | < 2.5 |

Table 5 shows particle and SDS recovery determined in the filtrate fraction of ultrafiltration of PAEC, free SDS or a mixture of SDS and a cationic polymer DA002. Particle recovery has been estimated from the mean light scattering of the permeate fraction of ultrafiltration. The particle recovery percentage was measured as the mean and SD of light scattering count rate of 3 measurements; the SDS recovery percentage was measured as mean and SD of 2 HPLC analysis.

Figures 10A and 10B show TEM micrography (5.000 x) of PAECs made from either CpG / P24 (20 / 20 µg/mL) (Figure 11A) or CpG / P24 (20 / 20 µg/mL + SDS 20 µg/mL) (Figure 11B). Figures 11A and 11B illustrate the morphology of the PAECs and confirm the nano-size range of these particles as observed by DLS, although they were mostly under an aggregated state which could originate from the drying required for electron microscopy observation. The images show that more homogeneous and smaller nanoparticles were produced when the ionic polymer was mixed with the surfactant SDS. (Figure 11B).

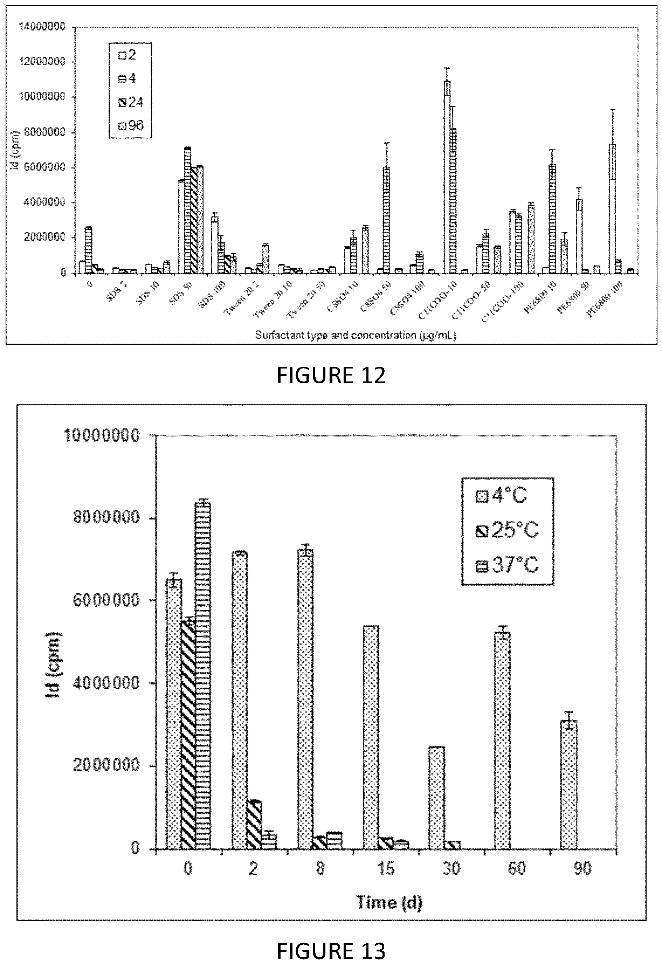

The release rate of the protein P24 and CpG was estimated *in vitro* in an indirect way, by following the DLS signal of the PAEC suspensions at different temperatures. The comparison of the DLS data summarized in Figure 13 shows that if the PAEC suspensions are stable at 4 and 20°C, their photocorrelation signal is rapidly lost when they are thermostatized at 37°C. This sharp decrease in mean light scattering intensity correlated with a large increase in polydispersity index of the nanodispersions, the apparition of a noisy autocorrelation curves and without any apparition of sediments in the medium, are strongly supporting the hypothesis of a dissociation of the PAECs when they are incubated at 37°C. The release rate is therefore occurring in the range of the body temperature. In particular, Figure 13 shows an analysis of the medium term stability of the PAEC loaded with CpG and P24 in a PBS medium at temperatures of 4, 25 or 37°C. The evolution with time of the mean light scattering Intensity has been analyzed up to 90 days.

### Example 5. Preparation of quaternary polyelectrolyte complexes with vaccine protein (P24), an oligonucleotide adjuvant (CpG) and different surfactants

Other surfactants than SDS have been evaluated for their potency to promote formation of PAECs and assure their stability. Following groups were tested:
- a family of aliphatic compounds of chemical structure homologous to SDS but differing either in the total number of carbons or in the nature of the anionic group (carboxylic group)
- sodium salts of cholic derivates, i.e.: sodium salt of cholic acid, taurochic acid or deoxycholic acid;
- non-ionic surfactants: Pluronic PE 6800, Pluronic F127, Tween 20 and Tween 85

The preparation of these PAECs was performed according to example 1, adopting the respective concentrations of P24 / CpG / surfactant / ionic polymer (DA002) of 50 / 20 / 2 to 100 / 90 (µg/mL).

The overtime monitoring of PAEC formation by DLS analysis in the presence of these different amphiphilic molecules has allowed us to constitute three groups:
Group 1: surfactants which provide formulations with very low count rate (background noise level or close). To this group, belong: - the more hydrophilic surfactants of the carboxylic acid family, i.e.: C₆-COOH and Cg-COOH, carboxylic acid salt with the shorter chain namely C₁₀-COO⁻ , Pluronic F127, the cholate family, samples Chol, D-Chol (deoxycholate), and T-Chol (taurocholate);
Group 2: surfactants which provide PAECs whose characteristics improve (size and concentration) with concentration, i.e. higher surfactant concentration enhancing PEG formation. This group include anionic surfactants C₁₂-COO⁻ and C₁₂-SO₄²⁻, but also polymeric amphiphilic surfactants such as Pluronic PE6800; and
Group 3: surfactants which favour PAEC formation with better results achieved at lower concentration. This observation has been noticed for the anionic surfactant C₁₈-SO₄²⁻ and the neutral surfactants Tween 20 and Tween 85.

The results are illustrated in Figure 12 which shows a comparison of the mean light scattering intensity (Id) measured by DLS of PAECs loaded with respective concentrations of P24/CpG/surfactant/polymer were 50 / 20 / 2 to 100 / 90 µg/mL and in function of the surfactant type and of their concentration in the quaternary complexes.

If the cholate derivatives are excluded from the analysis, the evolution in the property of these surfactants to enhance PAEC formation and to stabilize them is clearly affected by the HLB of these surface proteinaceous agents. Indeed:
- below a given aliphatic length of 12 carbons, the anionic alkyl surfactants seem to be too hydrophilic to promote the NP formation.
- below a HLB of 14.8, when we reach the critical alkyl chain length of 12 carbons, PAEC formation is promoted, but not with the same extent as observed in the presence of the anionic sulphate binding site present in SDS.
- for more hydrophobic compounds which have low CMC, PAEC formation is promoted at the lowest concentration of the surfactants. Indeed, CMC values of C₁₈-SO₄²⁻ and of Tween 20 are respectively about 6 times and 100 times lower than SDS. Accordingly, very low concentration of these two surfactants are required to produce stable PAECs.

All these observations support the hypothesis that PAEC formation and their stability are directly dictated to the CMC and HLB of this surfactant family. Moreover, this systematic study highlights that PAEC formation is also promoted in the presence of a non-ionic surfactant (such as Tween 20, Tween 85 and Pluronic F68). This observation is therefore indicative that PAEC formation can rely only on hydrophobic or hydrogen bondings and not necessarily from ionic interaction between the polycation / protein and the surfactant.

### Example 6. Encapsulation of PAECs loaded with human insulin within alginate beads.

To prepare an oral composition of PAECs, the PAECs may need to be protected from the gastric environment. To facilitate their transfer and release in the intestine, they have been immobilized in alginate microparticles of a size adaptable between at least 500 to 1000 µm. The PAECs have been first prepared according to previous examples in order to achieve concentration of human insulin of 150 µg/mL, of ionic polymer (DA002) 300 µg/mL, with or without SDS (150 µg/mL).

The encapsulation of the PAEC suspension in alginate is realized by the following steps:
1. 0.5 mL of 4wt% sodium alginate solution is mixed with an equivalent volume of each of the solutions of PAEC;
2. This mixture is sprayed at a flow-rate of 0.2 mL/min within 25 ml of a 2% CaCl₂ solution;
3. The Ca-alginate microparticles formed were washed three times by decantation within 0.9% NaCl; and
4. The microparticles are stored in 2mL of buffer containing NaN₃ (0.01%) and are stored at 4°C until future use.

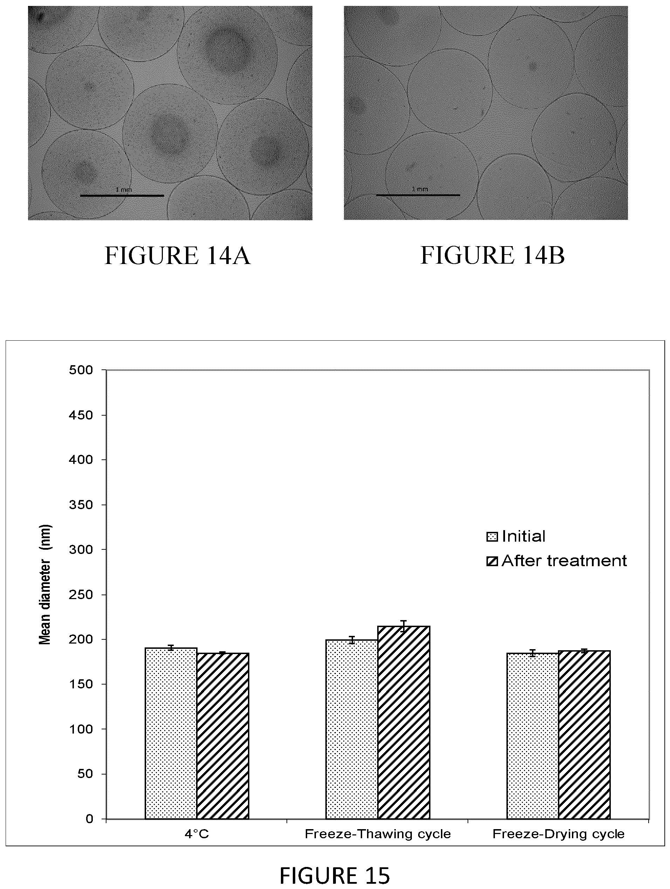

To assess the efficiency of this formulation step, the morphology of the microparticles was observed under optical transmission (see Figures 13A and B). Figure 14A shows a TEM micrography image (5.000 x) of alginate microbeads loaded with binary PAEC. Figure 14B shows a TEM micrography image (5.000 x) of alginate microbeads loaded with ternary PAEC stabilized with SDS. There is a difference of optical aspect between the two alginate beads loaded with two different batches of PAECs. For microparticles loaded with PAECs prepared without SDS, the presence of numerous particles entrapped within the beads are well visible under optical microscopy (Figure 14A), thus with a size well above their initial mean particle diameter (i.e. 200 nm). In contrast, for microparticles loaded with PAEC stabilized with SDS, no microaggregates are noticed within the alginate beads (Figure 14A).

The efficiency of release of the PAECs has also been determined after redissolution of the microbeads in a PBS medium containing EDTA, which is a calcium complexation agent able to trigger the rapid solubilisation of the beads. The DLS analysis of the particles released in the medium shows the release of the PAEC in the medium on a time scale of a few minutes.

### Example 7. Preparation of Amphiphilic Electrolyte Complexes (PAEC) made of CpG/P24/SDS/DA002 in view to compare their stability

Preparation of Amphiphilic Electrolyte Complexes (PAEC) made of CpG/P24/SDS/DA002 with a view to compare their stability on 1 day period according to the following three experimental conditions:
- storage at 4°C under the form of suspension;
- storage at -20°C; and
- storage at room temperature after lyophilisation.

PAEC have been made combining the antigenic protein P24, the oligonucleotide CpG, SDS and the polymer DA002 according to the protocol outlined in Example 4 to produce formulations with a composition identical to those assessed as reported in Example 6.

The mean size of three batches of PAEC, prepared using the same composition and same procedure has been controlled by DLS just after preparation and after their storage either:
- in suspension at 4°C; or
- after freezing imposed after PAEC preparation, storage at -20°C and thawing 1 day after; or
- after a freeze-drying cycle imposed after PAEC preparation, storage at 20°C and redispersion in an aqueous medium.

As disclosed on Figure 15, no significant difference in the mean size of the PAEC can be noticed whatever their mode of conservation. After lyophilisation, these PAEC are instantaneously dispersed using an aqueous phase. Accordingly, these PAEC can be prepared and stored under different physical forms, liquid suspension, frozen state or dried state in function of the final wishes of the end-users.

Figure 15 shows a comparison of the mean size of three batches of PAEC made of CpG/P24/SDS/DA002 which have been prepared using the same composition and same procedure as given in Examples 4 and 6 either just after preparation of the PAEC (Initial) and after their storage either: in suspension at 4°C, series labelled "4°C"; or after freezing imposed after PAEC preparation, storage at -20°C and thawing 1 day after, series labelled "Freeze-Thawing cycle"; or after a freeze-drying cycle imposed after PAEC preparation, storage at 20°C and redispersion in an aqueous medium, series labelled "Freeze-Drying cycle".

### Summary of characteristics

The examples have shown the following characteristics:
- rapid kinetics of PAEC formation (30 min);
- the mean size of the PAEC can be from 150 - 500 nm;
- the maximum loading capacity of the at least one proteinaceous agent can be in the range of up to 250 µg/mL for P24 and 80 µg/mL of CpG, but can be raised until 2 mg/mL in the case of human insulin;
- the PAEC are stable in vitro at 4°C for at least 2 weeks;
- the proteinaceous agent-containing PAEC are resistant to at least one freeze-thawing cycle and to a drying process, such as lyophilisation or spray-drying;
- the release rate of the proteinaceous agent(s) may be a function of the temperature and is significantly enhanced around the body temperature.
- an anionic surfactant can be largely associated to the PAEC (more than 97.5 wt %) through physically association, mainly internalized. The presence of a surfactant with amphiphilic properties could facilitate the diffusion of the PAEC through biological barriers by acting as a permeation enhancer;
- an optional surfactant may be amphiphilic with an adapted Hydrophilic/Lipophilic Balance to promote the nucleation of PAEC formation. Sodium dodecyl sulphate has proved to be convenient for the nanoencapsulation of human insulin and P24/CpG. Other amphiphilic compounds have proved to be also successful, including non-ionic amphiphilic compounds;
- the PAEC have a positive Zeta potential which can be adjusted to some extent by adjusting the content of the anionic excipient;
- the conformation state of the immobilized biopharmaceutic drug within the PAEC is not affected;
- the PAEC are cytocompatible and hemocompatible; and
- the PAEC can be loaded within gastroprotective microparticles.

## Claims

1. A complex comprising at least one proteinaceous agent, an ionic polymer comprising a repetitive unit of formula (I): 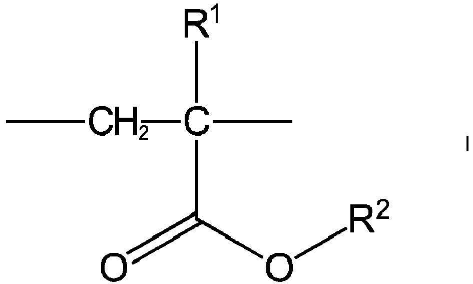 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which has a positive charge at a physiological pH; and optionally a surfactant; with the proviso that when the at least one proteinaceous agent is heparin and when the complex does not include a surfactant, the ionic polymer is not a poly (dimethyl amino) ethyl methacrylate having a relative number-average molecular weight of 8,000 or 15,000.

2. A complex as defined in Claim 1 which comprises a surfactant; preferably the surfactant is amphiphilic; preferably the surfactant is sodium dodecyl sulphate.

3. A complex as defined in Claim 1 or Claim 2 wherein the ionic polymer comprises a single repetitive unit of formula (I), or the repetitive unit of formula (I) is combined with other monomers or other polymer or oligomeric sequences.

4. A complex as defined in any one of the preceding Claims wherein the ionic polymer comprises repetitive units of formula (I) wherein at least some repetitive units of formula (I) are charged.

5. A complex as defined in any one of the preceding Claims wherein the ionic polymer is an ionic polymer of formula: 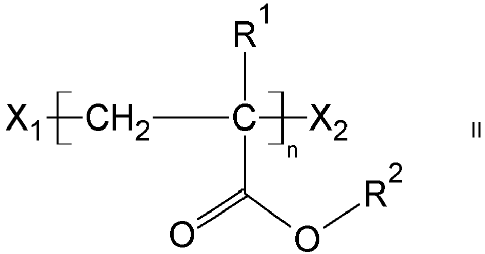 wherein X₁ and X₂ each respectively represent the alpha and omega end groups of the polymer; R¹ and R² are each as defined in Claim 1; and n represents the number of repetitive units of the ionic polymer.

6. A complex as defined in any one of the preceding Claims wherein the ionic polymer has a mean pKa of from 6 to 8.

7. A complex as defined in any one of the preceding Claims wherein the repetitive unit of formula (I) comprises N,N dimethyl amino ethyl methacrylate.

8. A complex as defined in any one of the preceding Claims wherein the at least one proteinaceous agent comprises one or more proteinaceous agents.

9. A complex as defined in any one of the preceding Claims wherein the at least one proteinaceous agent comprises an antigen; preferably the complex further comprises an adjuvant for improving immune response.

10. A complex as defined in any one of the preceding Claims which is encapsulated by an alginate coating.

11. A complex as defined in any one of the preceding Claims wherein the molar ratio of (dimethyl amino) ethyl methacrylate to a different repetitive unit of formula (I) is greater than 50%; preferably the molar ratio of (dimethyl amino) ethyl methacrylate to a different repetitive unit of formula (I) wherein R¹ represents a straight or branched chain alkyl group comprising from 1 to 6 carbon atoms is greater than 50%.

12. A complex for use in a method of medical treatment wherein the complex comprises at least one medically or veterinary active proteinaceous agent, an ionic polymer comprising a repetitive unit of formula (I): 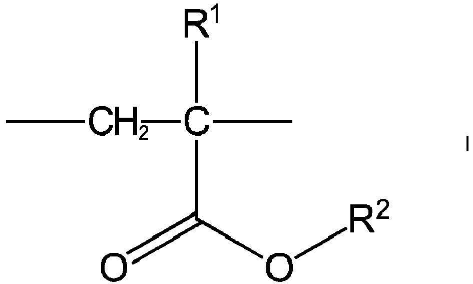 wherein R¹ represents a hydrogen atom or a straight or branched chain alkyl group, preferably a straight or branched chain alkyl group comprising from 1 to 6 carbon atoms, preferably a methyl group; R² represents a straight or branched chain alkyl group which is substituted by a group which has a positive charge at a physiological pH; or a copolymer thereof; and optionally a surfactant; preferably the complex is as defined in any one of claims 2 to 11.

13. A pharmaceutical composition comprising a pharmaceutically acceptable carrier and a complex as defined in any one of claims 1 to 11.

14. A pharmaceutical composition for use in a method of medical or veterinary treatment wherein the composition comprises a pharmaceutically acceptable carrier and a complex as defined in claim 12; preferably the complex is as defined in any one of claims 2 to 11.

15. A method of medical treatment which method includes a step of administering to a human or animal in need of such treatment an effective amount of a complex which comprises at least one medically active proteinaceous agent, an ionic polymer comprising a repetitive unit of formula (I) as defined in Claim 12 or a copolymer thereof; and optionally a surfactant; preferably the complex is as defined in any one of claims 2 to 11.
